# EUROPEAN PATENT APPLICATION

(11) **EP 1 541 544 A1**
(43) Date of publication of application: **15.06.2005**
(21) Application number: 03738140.7
(22) Date of filing: 02.07.2003
(51) Int. Cl.: C07C 69/007, C07C 69/025, C07C 69/16, A61K 31/222, A61K 7/00, A61P 3/02

(54) **METHOD OF PREPARING HYDROXYTYROSOL ESTERS, ESTERS THUS OBTAINED AND USE OF SAME**

(30) Priority: 03.07.2002 ES 200201554
(71) Applicant: CONSEJO SUPERIOR DE INVESTIGACIONES CIENTIFICAS, 28006 Madrid (ES); Universidad de Sevilla, 41013 Sevilla (ES)
(72) Inventor: ALCUDIA GONZALEZ, Felipe, 41013 SEVILLA (ES); CERT VENTUL , Arturo, Insto. Grasa, 41012 SEVILLA (ES); ESPARTERO SANCHEZ, José Luis, 41013 SEVILLA (ES); MATEO BRIZ, Raquel, Insto. Grasa, 41012 SEVILLA (ES); TRUJILLO PEREZ-LANZAC, Mariana, 41013 SEVILLA (ES)
(74) Representative: Ungria Lopez, Javier
(86) International application number: PCT/ES2003/000327
(87) International publication number: WO 2004/005237

(57) **Abstract**

The invention relates to a method of preparing a wide range of hydroxytyrosol esters. According to the invention, the compounds are synthesised by reacting synthetic hydroxytyrosol or natural hydroxytyrosol compounds, oleuropein or oleuropein aglycones (which are found in olive oil, vegetable water, olive marc or olive leaves) with an acylating agent which is a compound containing at least one acylic group R, wherein R is H, an alkyl radical with between 1 and 31 linear, branched or cyclic carbon atoms, an alkenyl radical with up to 31 linear, branched or cyclic carbon atoms or an aryl group. The invention also relates to the esters prepared with the inventive method, which can be used as an additive in food and cosmetic products as well as in pharmaceutical preparations.

## Description

### OBJECT OF THE INVENTION

The process object of the present invention provides a simple and effective procedure for the preparation of a wide range of hydroxytyrosol esters of general formula:

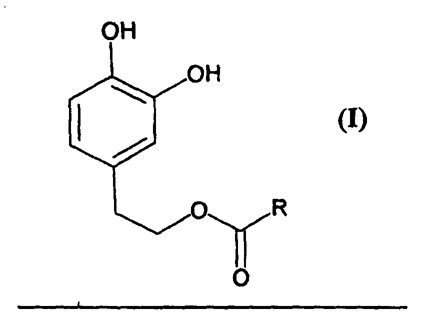

The synthesis of the compounds is carried out by reacting synthetic hydroxytyrosol or natural hydroxytyrosol compounds, oleuropein or oleuropein aglycones (which are found in olive oil, vegetable water, olive marc or olive leaves) with an acylating agent which is an organic acid or an ester containing at least one acyl group R, wherein R is H, an alkyl radical with between 1 and 21 linear or branched carbon atoms, an alkenyl radical with up to 21 carbon atoms or an aryl group. The esters prepared with the process of the invention can be used as an additive in food and cosmetic products as well as in pharmaceutical preparations.

### STATE OF THE ART

Antioxidants are added to oils and fats, as well as to food, in order to prevent the formation of undesirable colours and flavours and of other compounds originating in the oxidation of lipids ["Food Antioxidants", B.J.F. HUDSON ed., Elsevier, London, 1990; G. PASCAL in "Aditivos y auxiliares de fabricación en las industrias agroalimentarias", L.J. Multon ed., Ed. Acribia, Zaragoza (Spain), 1988, pp. 157 et seq.]. It is estimated that the useful life of many food products increases by between 15 and 200 % due to the use of antioxidants [R. MAESTRO DURÁN AND R. BORJA PADILLA, Grasas y Aceites, 44 (1993), 101].
A wide range of antioxidant additives is currently used in the preparation of food products [Royal Decree 145/1997, Official State Gazette of 22/3/97, pp. 9378 et seq.] and cosmetics ["Inventario de Ingredientes Cosméticos", Publications Centre of the Ministry of Health and Consumption, Madrid, 1996], of both natural and , synthetic origin, depending on the nature, to a greater or lesser lipid degree, of the product it is sought to protect from oxidation.
Standing out among natural antioxidants for their high activity are polyphenolic compounds, above all the ortho and paracatechols. Hydroxytyrosol (II) is one such o-catechol, and is found in various natural sources, with its presence in olive being especially important [A. VÁZQUEZ RONCERO, Rev. Fr. Corps Gras, 25 (1978) 21], whether free or in the form of derivatives (fundamentally oleuropein, III, [L.M. PANIZZI, Gazz. Chim. Ital., 90 (1960) 1449]). Hydroxytyrosol displays a protective capacity against oxidation that is much greater than the antioxidant additives normally used in the conversion of fatty food products: tocopherols (antioxidants of natural origin) and butyl hydroxytoluol (BHT, an antioxidant of synthetic origin) [M. SERVILI, et al., Rev. Ital.

Sostanze Grasse, 73 (1996) 55]. Nevertheless, it cannot be used for this type of food as it is virtually insoluble in lipophilic medium

However, it is known that virgin olive oil (VOO) maintains certain levels of oleuropein aglycones (IV and V) which have a bitter taste, which they impart to the oil [G.F. MONTEDORO et al., J. Agric. Food Chem., 415 (1993) 2228; A. VÁZQUEZ RONCERO et al., Grasas y Aceites, 25 (1974) 269]. These derivatives (IV and V) possess an antioxidant activity similar to that displayed by free hydroxytyrosol [M. SERVILI et al.; Rev. Ital. Sostanze Grasse, 73 (1996) 55], which indicates that the presence of an ester bond in the molecule does not affect the antioxidant power.

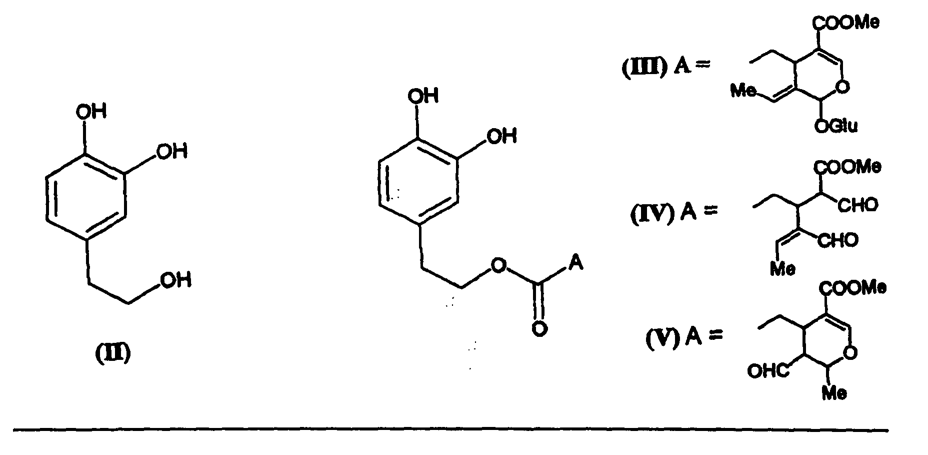

The presence of another hydroxytyrosol ester has recently been detected in VOO: hydroxytyrosol acetate (VI) [M. BRENES et al., J. Agric. Food Chem., 47 (1999) 3535; J.L. ESPARTERO et al., Libro de Resúmenes de la XXVII Reunión Bienal de la Real Sociedad Espanola de Quimica, Tenerife, 1999, Ref. S6-1C-10 p. 165; R. MATEOS et al., J. Agric.

Food Chem. 49 (2001) 2185]. This compound has been subjected to studies in order to confirm its antioxidant capacity, with the result that it has the same activity towards oxidation as does free hydroxytyrosol itself. Moreover, it is liposoluble and is not bitter, characteristics which make it especially attractive for its possible use as a food additive.

Moreover, it has been suspected for some time that the phenols contained in olive oil have a beneficial effect on human health [F. VISIOLI and C. GALLI, J. Agric. Food Chem. 46 (1998) 4292]. It has been seen that hydroxytyrosol prevents oxidative damage to DNA and oxidation of low density lipoproteins [O.I. ARUOMA et al., J. Agric. Food Chem., 46 (1998) 5181], it inhibits aggregation of platelets [A. PETRONI et al., Thromb. Res. 78 (1995) 151], it inhibits the activity of certain lipoxygenases [N. KOHYAMA et al., Biosci., Biotechnol., Biochem. 61 (1997) 347; R. De la PUERTA et al., Biocherm. Pharmacol. 57 (1999) 445], and it is absorbed by the human organism in the ingestion of olive oil [F. VISIOLI et al., FEBS Lett., 468 (2000) 159]. All these studies suggest that hydroxytyrosol and its derivatives can have a pharmacological use.

A reference on the preparation of a hydroxytyrosol ester was published by Baraldi in 1983 [P.G. BARALDI et al., Liebigs Ann. Chem., 83 (1983) 684]. In this case, hydroxytyrosol acetates was obtained by means of a synthetic route, previously investigated by Schöpf [C. SCHÖPF et al., Liebigs Ann. Chem., 563 (1949) 86], which involved five stages starting from 2-(3,4-dimethoxyphenyl)ethanol (VII) as shown in the following 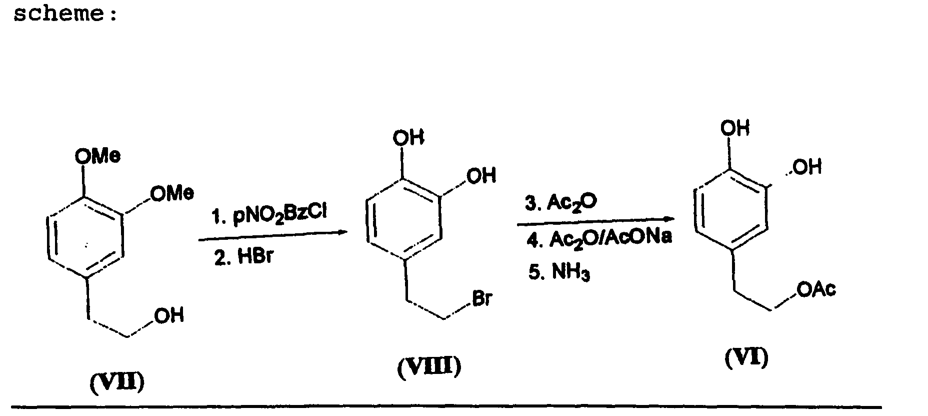 A new synthesis has recently been described [M.H. GORDON et al., J. Agric. Food Chem., 49 (2001) 2480], starting from hydroxytyrosol and consisting of three steps: treatment with benzyl bromide in order to give 2-(3,4-dibenzyloxyphenyl)ethanol, acetylation with acetic acid in pyridine in order to obtain the acetate of the above product, and finally, elimination of the benzyl groups by hydrogenation with a palladium catalyst.

Both procedures are lengthy, some of the stages require special conditions and/or especially careful handling in order to prevent unnecessary losses of product and total yields are low. By means of these procedures, moreover, only the said acetate of hydroxytyrosol (VI) can be synthesised.

### EXPLANATION OF THE INVENTION

The aim of the present invention is a process for the preparation of hydroxytyrosol esters of general formula:

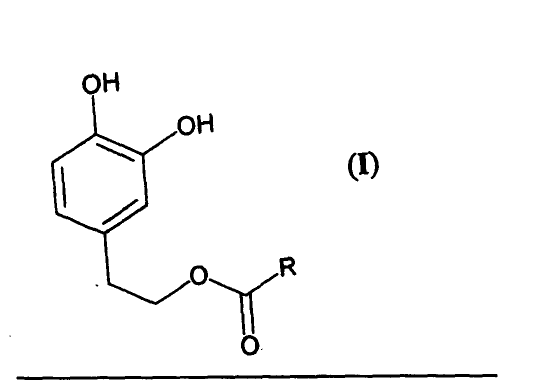

The process is a regioselective esterification reaction consisting of the following stages:
a) contact between hydroxytyrosol or natural esters of hydroxytyrosol (oleuropein, oleuropein aglycones) and an acylating agent in a concentration ratio of between 10:1 and 1.1000 (hydroxytyrosol / acylating agent), at a temperature between 0 and 150 °C during a period of time between 30 minutes and 1 month, in the presence of a catalyst.
b) isolation and purification of the hydroxytyrosol esters obtained in the above stage.
The acylating agent is an organic acid or ester containing at least one acyl group R. The radical R of the general formula can be H, an alkyl radical, an alkenyl radical or an aryl group. The alkyl radical can be any radical derived from a linear or branched chain alkane up to 21 carbon atoms both included. The alkenyl radical can be any radical derived from a linear or branched chain alkene of up to 21 carbon atoms inclusive, having one or more degrees of unsaturation in any position of the chain. The aryl radical is a phenyl group or substituted derivative thereof.

An acidic catalyst is used if the acylating agent is an organic acid, and an acidic or enzymatic catalyst if the acylating agent is an ester. In the case of the acylating agent being an ester, the reaction is regioselective by at least 95%.
The reaction is conducted in the presence of an inert solvent, and an acidic or enzymatic catalyst. The solvent is selected from among any of the following: esters, ethers or halogenated hydrocarbons.
The catalyst can be a mineral acid, phosphoric acid, an organylsulphonic acid or a lipase. In the presence of sulphuric acid, the reaction takes place at ambient temperature during a period of time between 30 minutes and 24 hours.
The process object of the invention can include, following the contact stage, the isolation and purification of the hydroxytyrosol esters obtained.
The hydroxytyrosol esters obtained by means of the invention process can be used as additives in food formulations, in cosmetic products and in pharmaceutical preparations.
The products of the reaction obtained can, without any further isolation of the esters, be used as additives in the said applications without prior purification.
In particular, for use in food formulations, the esters can be added alone or together with other natural antioxidants of the orthodiphenolic or diterpenic type, in a quantity such that their total concentration in the apolar phase of the food does not exceed 200 ppm.
Also constituting an object of this invention is a food formulation containing as additive hydroxytyrosol esters obtained by means of the invention process, alone or together with other natural oxidants of the orthophenolic or diterpenic type, in a quantity such that their total concentration in the apolar phase of the food does not exceed 200 ppm.
Finally, likewise an object of this invention is a cosmetic product and a pharmaceutical preparation comprising hydroxytyrosol esters obtained by means of the invention process.

### DESCRIPTION OF THE INVENTION

This invention describes a new process for the preparation of hydroxytyrosol esters [2-(3,4-dihydroxyphenyl)ethanol] of general formula (I) 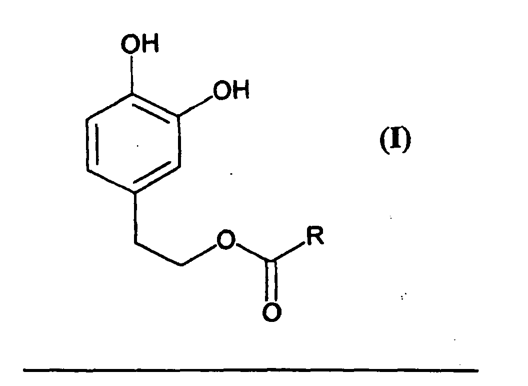 wherein R is H, an alkyl radical of between 1 and 21 carbon atoms, either linear or branched, an alkenyl radical of up to 21 carbons atoms, or an aryl group
starting from hydroxytyrosol or natural esters of hydroxytyrosol (oleuropein, oleuropein aglycones) and an acylating agent which is an organic acid or an ester containing at least one acyl group R. The hydroxytyrosol can have a synthetic origin [R. CAPASSO et al., J. Agric. Food Chem., 47 (1999) 1745; C. BAI et al., J. Agric. Food Chem., 46 (1998) 3998; R. VERHE et al., Bull. Liaison Groupe Polyphenols, 15 (1992) 237; A. BIANCO et al., Synth. Comun. 18 (1988) 1765; P.G. BARALDI et al., Liebigs Ann. Chem., 83 (1983) 684; J.C. SPIN et al., J. Agric. Food Chem., 49 (2001) 1187] or a natural one. The oleuropein and oleuropein aglycones are of natural origin. Both the hydroxytyrosol and the aleuropein and its aglycones can be contained in the olive fruit, the olive oil, the olive leaves and in waste products from the preparation of olive oil or of table olives, basically in vegetable water, in olive marc and in the wash water obtained during the preparation of Spanish style green olives [M.J. AMIOT et al., J. Agric. Food Chem., 34 (1986) 823; R. BRIANTE et al., J. of Biotechnol., 93 (2002) 109; M. BRENES et al., J. Agric. Food Chem., 43 (1995) 2702; R. MATEOS et al., J. Agric. Food Chem., 49 (2001) 2185; R. CAPASSO et al., J. Agric. Food Chem., 47 (1999) 1745; R. CAPASSO et al., Agrochimica, 38 (1994) 165; R. CAPASSO et al., Phytochemistry, 12 (1992) 4125; E. RAGAZZI et al., Ann. Chim., 57 (1967) 1476] and R. CAPASSO et al., Appl. Biochem. Biotechnol., 60 (1996) 365; L.M. PANIZZI et al., Gazz. Chim. Ital. 90 (1960) 1449].

The term "alkyl radical of between 1 and 21 carbon atoms" must, in the sense used in this description, be understood as any radical derived from a linear or branched chain alkane of up to 21 carbon atoms inclusive.

Analogously, the term "alkenyl radical of between 1 and 21 carbon atoms" must be understood as any radical derived from a linear or branched chain alkene of up to 21 carbon atoms inclusive, having one or more degrees of non-saturation in any position of the chain.

In a similar manner, the term "aryl" must be understood as a phenyl group or a substituted benzene derivative with one or more groups of whatsoever nature.

The synthesis of the compounds (I) object of this invention is carried out by reaction of synthetic hydroxytyrosol (II) (obtained by any of the processs described earlier) or of the natural products: hydroxytyrosol (II), oleuropein (III) and oleuropein aglycones (IV and V) (derived from any of the sources mentioned earlier), with an organic acid or an ester, wherein R has the meaning stated earlier. This reaction can be carried out either with heating or at ambient temperature, and in the presence of an acidic catalyst if the acylating agent is an organic acid, and of an acidic or enzymatic catalyst if the acylating agent is an ester, according to the following scheme:

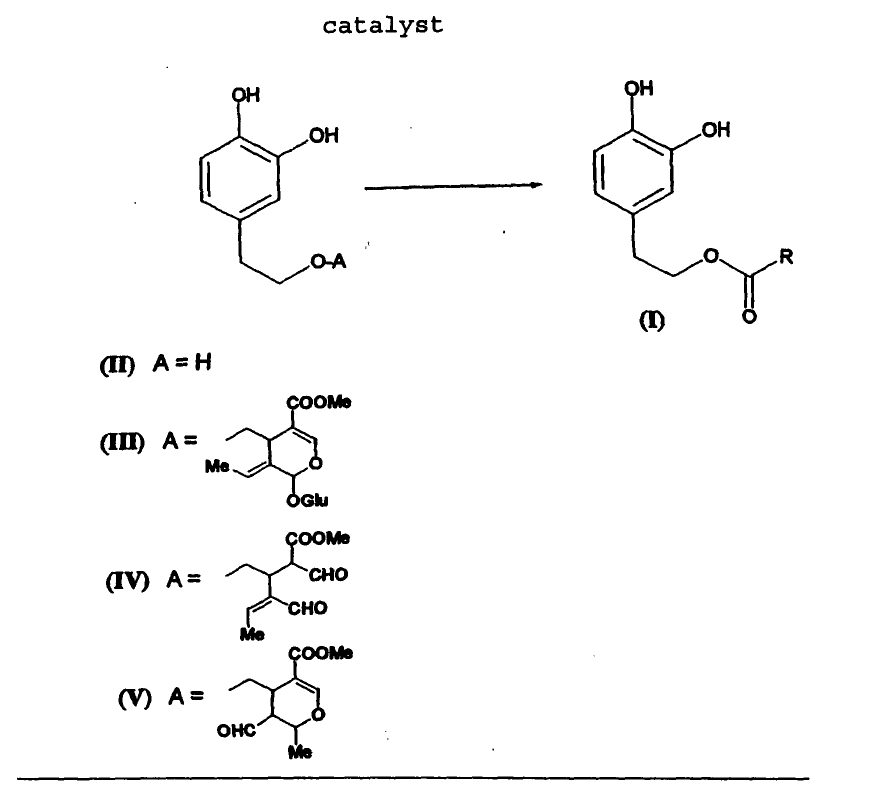

As acidic catalyst, any substance of an acidic nature can be used, preferably: sulphuric acid (H₂SO₄), hydrogen chloride (HC1), phosphoric acid, trifluoroacetic acid (CF₃COOH), acetic acid (CH₃COOH), p-toluenesulphonic acid (CH3C6H4SO3H) or camphorsulphonic acid (C10H16HO4S). As enzymatic catalyst, any enzyme with esterase or lipase activity, raw or purified, can be used, preferably porcine pancreatic lipase (PPL), papain, or horse, pig, cow, rabbit or sheep liver esterase.
The reaction can be conducted in the absence or presence of solvents that are inert.
The hydroxytyrosol esters obtained by means of the process of the invention have been subjected to studies in order to check their antioxidant capacity, with the result of having the same activity towards oxidation as does free hydroxytyrosol itself. The advantage they show compared to the latter is that they are much more soluble in lipid environments (oils, lards, fats, etc.). These compounds of formula (I) are therefore useful for the food and cosmetic industry as antioxidant additives. Moreover, hydroxytyrosol esters possess pharmacological effects similar to those observed with hydroxytyrosol, which can be greater owing the liposoluble nature of these molecules.

With respect to the state of the prior art, the process of the invention presents the following advantages:
1) It is the first time that the preparation of hydroxytyrosol esters by means of a transesterification reaction has been described.
2) Catalysts are used that can be easily eliminated, thereby avoiding the need for a later purification process.
3) By means of the invention process, any hydroxytyrosol ester can in principal be synthesised simply by changing the nature of the RCOX reagent.
4) The reaction takes place with high chemical yields.
5) It is a simpler and more efficacious process than that described in the state of the art for the synthesis of hydroxytyrosol acetate.
6) The hydroxytyrosol esters obtained by means of the invention process are highly soluble in lipids and have no bitter taste.
7) Their antioxidant activity is greater than that of other additives currently used (tocopherols and BHT).
8) They are prepared starting from hydroxytyrosol or from the natural derivatives of hydroxytyrosol mentioned earlier, which are found in abundance in the waste-waters from the production of olive oil (vegetable water), in the solid waste (marc) and in olive leaves, from where they can be extracted by known processs. This would permit exploitation of the waste products from olive presses.
9) It is easy to prepare hydroxytyrosol esters whose radical R is a fatty acid present naturally in animal fats and/or vegetable oils (palmitic, stearic, oleic, linoleic, etc.), which makes them candidates for being used as antioxidant additives in human food and in pharmacological applications.

### MODE OF EMBODIMENT OF THE INVENTION

The invention is illustrated in the following examples, which in no way aim to limit the scope of the present invention

### Example 1: Preparation of 2-(3,4-dihydroxyphenyl)ethyl acetate (Hydroxytyrosol acetate)

### Method A: Starting from hydroxytyrosol

50 mg of raw porcine pancreatic lipase (Aldrich) are added to a solution of 50 mg of hydroxytyrosol (II) in 2 mL of ethyl acetate and the resulting mixture is stirred for 48 hours. The resulting suspension is filtered through celite and the solvent is evaporated in vacuo, with pure hydroxytyrosol acetate being obtained, with a yield of 86%.

### Method B: Starting from oleuropein

0.4 mL of 12N hydrochloric acid is added to a solution of 50 mg of oleuropein (III) in 4 mL of ethyl acetate and the resulting mixture is stirred for 16 hours at a temperature of 40°C. The resulting suspension is washed with a saturated solution of sodium bicarbonate, dried over anhydrous sodium sulphate, filtered and evaporated to residue. The product is purified by column chromatography with pure hydroxytyrosol acetate being obtained, with a yield of 72%.
Nuclear Magnetic Resonance Spectroscopy (NMR)
¹H NMR (DMSO-d₆) δ : 6.63 (d, 1H), 6.60 (d, 1H), 6.46 (dd, 1H), 4.10 (t, 2H), 2.68 (t, 2H), 1.97 (s, 3H) ppm.
¹³C (DMSO-d₆) δ :170.2, 145.0, 143.7, 128.5, 119.4, 116.1, 115.5, 64.6, 33.5, 20.6 ppm
Mass Spectrometry (MS)
Calculated for C10H13O4 [M+H]+ : 197.081384
Determined by HRMS (Cl) : 197.080136 (6.3 ppm)

| Element analysis | | |
|---|---|---|
| Calculated for C₁₀H₁₂O₄ | C, 76.59; | H, 10.64 |
| Found | C, 76.32; | H, 10.91 |

### Example 2: Preparation of 2-(3,4-dihydroxyphenyl)ethyl oleate (Hydroxytyrosol oleate)

### Method A: Starting from ethyl oleate

5 mg of p-toluenesulphonic acid (Aldrich) are added to a solution of 50 mg of hydroxytyrosol (II) in 1.0 mL of ethyl oleate and the resulting mixture is heated at 60 °C for 24 hours. After leaving it to cool, it is washed with a saturated solution of NaHCO3, the organic fraction is gathered, dried over anhydrous sodium sulphate and the mixture is introduced into a chromatography column for purification, with the pure hydroxytyrosol oleate being obtained, with a yield of 76%.
Nuclear Magnetic Resonance Spectroscopy (NMR)
¹H NMR (DMSO-d₆) δ : 6.62 (d, 1H), 6.59 (d, 1H), 6.44 (dd, 1H), 5.31 (m, 2H), 4.10 (t, 2H), 2.67 (t, 2H), 223 (t, 2H), 1.97 (q, 4H), 1.47 (m, 2H), 1.25 (m, 20H), 0.84 (t, 3H) ppm.
¹³C (DMSO-d₆) δ : 172.7, 145.0, 143.7, 129.6, 128.5, 119.3, 116.1, 115.4, 64.5, 33.7, 33.4, 31.2, 29.0, 28.9, 28.7, 28.6, 28.5, 28.4, 28.3, 26.5, 24.3, 22.0. 13.8 ppm
Mass Spectrometry (MS)
Calculated for C₂₆H₄₃O₄ [M+H]+ : 419.316135
Determined by HRMS (Cl) : 419.315427 (1.7 ppm)
Element analysis
Calculated for C₂₆H₄₂O₄ x 1/2 H₂O : C, 73.07; H, 10.07
Found : C, 73.58; H, 10.48

### Method B: Starting from olive oil

1 drop of sulphuric acid is added to a solution of 50 mg of hydroxytyrosol (II) in 0.5 mL of olive oil and the resulting mixture is stirred for 24 hours. The mixture is washed with a saturated solution of NaHCO₃, the organic fraction is gathered, dried over sodium sulphate and the solvent is evaporated. The mixture of fatty esters of hydroxytyrosol (fundamentally hydroxytyrosol oleate), diluted in the remaining olive oil, can be used without any need for purification. If it is wished to purify, this can be done by means of chromatography column, with a liquid mixture of hydroxytyrosol oleate (83%), hydroxytyrosol palmitate (11%) and hydroxytyrosol linoleate (6%) being obtained.

## Claims

1. Process for the preparation of hydroxytyrosol esters of general formula: 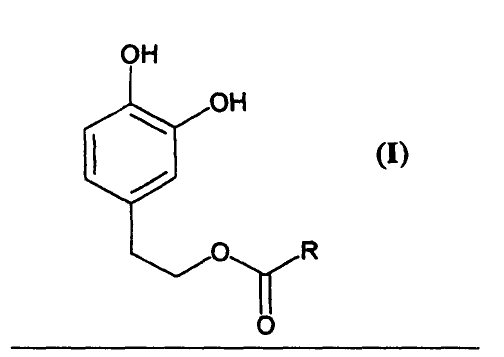 **characterised in that** said method is a regioselective esterification reaction carried out by means of contact among some of the following compounds
- hydroxytyrosol
- hydroxytyrosol as intermediate compound in the reduction of 3,4-dihydroxyphenyl acetic acid or any of its derivative esters
- oleuropein
- oleuropein aglycones
and an acylating agent in a concentration ratio of between 10:1 and 1:1000 (hydroxytyrosol or similar compound / acylating agent), at a temperature between 0 and 150 °C during a period of time between 30 minutes and 1 month, in the presence of a catalyst.

2. Process for the preparation of hydroxytyrosol esters according to claim 1, **characterised in that** the acylating agent is a compound containing at least one R radical acylic group.

3. Process for the preparation of hydroxytyrosol esters according to claims land 2, **characterised in that** the esterification carried out with an ester as acylating agent is regioselective by at least 95%.

4. Process for the preparation of hydroxytyrosol esters according to claims 1-3, **characterised in that** the radical R is H, an alkyl radical, an alkenyl radical or an aryl group.

5. Process for the preparation of hydroxytyrosol esters according to claims 1-4, **characterised in that** the alkyl radical can be any radical with linear, branched or cyclic carbon chain structure, substituted or not, of up to and comprising 31 carbon atoms.

6. Method for the preparation of hydroxytyrosol esters according to claims 1-4, **characterised in that** the alkenyl radical can be any radical with linear, branched or cyclic carbon chain structure, substituted or not, of up to and including 31 carbon atoms and which has one or more degrees of unsaturation in any position of the chain.

7. Process for the preparation of hydroxytyrosol esters according to claims 1-4, **characterised in that** the aryl radical is a phenyl group or a substituted derivative thereof.

8. Process for the preparation of hydroxytyrosol esters according to claims 1-7, **characterised in that** the reaction is optionally carried out in the presence of a solvent selected from among any of the following: esters, ethers or halogenated hydrocarbons.

9. Process for the preparation of hydroxytyrosol esters according to claims 1-8, **characterised in that** the reaction is carried out in the presence of an acidic or enzymatic catalyst.

10. Process for the preparation of hydroxytyrosol esters according to claim 9, **characterised in that** the catalyst is a mineral acid.

11. Process for the preparation of hydroxytyrosol esters according to claim 10, **characterised in that** the catalyst is phosphoric acid.

12. Process for the preparation of hydroxytyrosol esters according to claim 9, **characterised in that** the catalyst is an organylsulphonic acid.

13. Process for the preparation of hydroxytyrosol esters according to claim 9, **characterised in that** the catalyst is a lipase.

14. Process for the preparation of hydroxytyrosol esters according to claims 1-13, **characterised in that** it includes, after the contact stage, the isolation and purification of the hydroxytyrosol esters obtained.

15. Hydroxytyrosol esters obtainable by means of a process according to claims 1-14, **characterised in that** the acyl radical consists of a chain comprising from 3 to 6 carbon atoms.

16. Hydroxytyrosol esters obtainable by means of a process according to claims 1-14, **characterised in that** the acyl radical consists of a chain containing from 7 to 9 carbon atoms.

17. Hydroxytyrosol esters obtainable by means of a process according to claims 1-14, **characterised in that** the acyl radical consists of a chain containing more than 9 carbon atoms.

18. Use of hydroxytyrosol esters according to claims 15-17, as additive for food formulations.

19. Use of hydroxytyrosol esters as additive in food formulations according to claim 18, **characterised in that** the hydroxytyrosol esters are obtained by means of a method according to claims 1-14.

20. Use of hydroxytyrosol esters as additive in food formulations according to claims 18 and 19, **characterised in that** said esters are added alone or together with other natural antioxidants of the orthodiphenolic or diterpenic type, in a quantity such that their total concentration in the apolar phase of the food does not exceed 200 ppm.

21. Use of the mixture of products obtained in the reaction by means of a process according to claims 1-13, with prior elimination of the catalyst and of the solvent, as an additive in food formulations.

22. Use of hydroxytyrosol esters according to claims 15-17 as additive in cosmetic products.

23. Use of hydroxytyrosol esters as additive in cosmetic products according to claim 22, **characterised in that** the hydroxytyrosol esters are obtained by means of a process according to claims 1-14.

24. Use of the mixture of products obtained in the reaction by means of a process according to claims 1-13, with prior elimination of the catalyst and of the solvent, as an additive in cosmetic products.

25. Use of hydroxytyrosol esters as according to claims 15-17 in pharmaceutical preparations.

26. Use of hydroxytyrosol esters as additive in pharmaceutical preparations according to claim 25, **characterised in that** the hydroxytyrosol esters are obtained by means of a process according to claims 1-14.

27. Use of the mixture of products obtained in the reaction by means of a process according to claims 1-13, with prior elimination of the catalyst and of the solvent, in pharmaceutical preparations.

28. Food formulation which comprises as additive hydroxytyrosol esters according to claims 15-17, alone or together with other natural antioxidants of the orthodiphenolic or diterpenic type, in a quantity such that their total concentration in the apolar phase of the food does not exceed 200 ppm.

29. Cosmetic product comprising as additive hydroxytyrosol esters according to claims 15-17.

30. Pharmaceutical preparation comprising as additive hydroxytyrosol esters according to claims 15-17.

1. Process for the preparation of hydroxytyrosol esters of general formula: 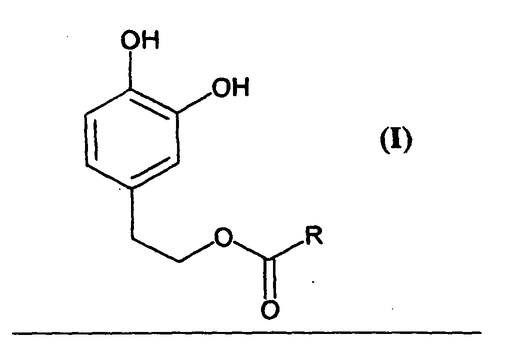 by means of a regioselective esterification reaction carried out by means of contact among some of the following compounds
- hydroxytyrosol
- hydroxytyrosol as intermediate compound in the reduction of 3,4-dihydroxyphenyl acetic acid or any of its derivative esters
- oleuropein
- oleuropein aglycones
and an acylating agent, **characterised in that** said acylating agent is an organic acid or an ester comprising at least one acyl group R and is used in a concentration ratio of between 10:1 and 1.1000 (hydroxytyrosol or similar compound / acylating agent), at a temperature between 0 and 150 °C during a period of time between 30 minutes and 1 month, in the presence of an acidic catalyst if the acylating agent is an organic acid, and of an acidic or enzymatic catalyst if the acylating agent is an ester.

2. Process for the preparation of hydroxytyrosol esters according to claim 1, **characterised in that** the esterification carried out with an ester as acylating agent is regioselective by at least 95%.

3. Process for the preparation of hydroxytyrosol esters according to claims 1-2, **characterised in that** the radical R is H, an alkyl radical, an alkenyl radical or an aryl group.

4. Process for the preparation of hydroxytyrosol esters according to claims 1-3, **characterised in that** the alkyl radical can be any radical with linear, branched or cyclic carbon chain structure, substituted or not, of up to 31 carbon atoms inclusive.

5. Process for the preparation of hydroxytyrosol esters according to claims 1-3, **characterised in that** the alkenyl radical can be any radical with linear, branched or cyclic carbon chain structure, substituted or not, of up to 31 carbon atoms inclusive, and which has one or more degrees of unsaturation in any position of the chain.

6. Process for the preparation of hydroxytyrosol esters according to claims 1-3, **characterised in that** the aryl radical is a phenyl group or a substituted derivative thereof.

7. Process for the preparation of hydroxytyrosol esters according to claims 1-6, **characterised in that** the acylating agent used is triglycerides contained in oils or fats.

8. Process for the preparation of hydroxytyrosol esters according to claims 1-7, **characterised in that** the reaction is optionally carried out in the presence of a solvent selected from among any of the following: esters, ethers or halogenated hydrocarbons.

9. Process for the preparation of hydroxytyrosol esters according to claims 1-8, **characterised in that** the catalyst is a mineral acid.

10. Process for the preparation of hydroxytyrosol esters according to claim 9, **characterised in that** the catalyst is phosphoric acid.

11. Process for the preparation of hydroxytyrosol esters according to claims 1-8, **characterised in that** the catalyst is an organylsulphonic acid.

12. Process for the preparation of hydroxytyrosol esters according to claims 1-8, **characterised in that** the catalyst is a lipase.

13. Process for the preparation of hydroxytyrosol esters according to claims 1-12, **characterised in that** it comprises, after the contact stage, the isolation and purification of the hydroxytyrosol esters obtained.

14. Hydroxytyrosol esters obtainable by means of a process according to claims 1-13, **characterised in that** the acyl radical consists of a chain containing from 3 to 6 carbon atoms.

15. Hydroxytyrosol esters obtainable by means of a process according to claims 1-13, **characterised in that** the acyl radical consists of a chain comprising from 7 to 9 carbon atoms.

16. Hydroxytyrosol esters obtainable by means of a process according to claims 1-13, **characterised in that** the acyl radical consists of a chain containing more than 9 carbon atoms.

17. Use of hydroxytyrosol esters according to claims 14 and 16, as additive for food formulations.

18. Use of hydroxytyrosol esters as additive in food formulations according to claim 18, **characterised in that** the hydroxytyrosol esters are obtained by means of a process according to claims 1-13.

19. Use of hydroxytyrosol esters according to claim 15, as additive for food formulations.

20. Use of hydroxytyrosol esters as additive in food formulations according to claims 17-19, **characterised in that** said esters are added alone or together with other natural antioxidants of the orthodiphenolic or diterpenic type, in a quantity such that their total concentration in the apolar phase of the food does not exceed 200 ppm.

21. Use of the mixture of products obtained in the reaction by means of a process according to claims 1-12, with prior elimination of the catalyst and of the solvent, as an additive in food formulations.

22. Use of hydroxytyrosol esters according to claims 14 and 16 as additive in cosmetic products.

23. Use of hydroxytyrosol esters as additive in cosmetic products according to claim 22, **characterised in that** the hydroxytyrosol esters are obtained by means of a process according to claims 1-13.

24. Use of hydroxytyrosol esters according to claim 15 as additive in cosmetic products.

25. Use of the mixture of products obtained in the reaction by means of a process according to claims 1-12, with prior elimination of the catalyst and of the solvent, as an additive in cosmetic products.

26. Use of hydroxytyrosol esters as according to claims 14 and 16 in pharmaceutical preparations.

27. Use of hydroxytyrosol esters as additive in pharmaceutical preparations according to claim 26, **characterised in that** the hydroxytyrosol esters are obtained by means of a process according to claims 1-13.

28. Use of hydroxytyrosol esters according to claim 15 in pharmaceutical preparations.

29. Use of the mixture of products obtained in the reaction by means of a process according to claims 1-12, with prior elimination of the catalyst and of the solvent, in pharmaceutical preparations.

30. Food formulation which comprises as additive hydroxytyrosol esters according to claims 14-16, alone or together with other natural antioxidants of the orthodiphenolic or diterpenic type, in a quantity such that their total concentration in the apolar phase of the food does not exceed 200 ppm.

31. Cosmetic product which comprises as additive hydroxytyrosol esters according to claims 14-16.

32. Pharmaceutical preparation which comprises as additive hydroxytyrosol esters according to claims 14-16.
